# EUROPÄISCHE PATENTSCHRIFT

(11) **EP 0 518 051 B1**
(45) Veröffentlichungstag und Bekanntmachung des Hinweises auf die Patenterteilung: **17.01.1996**
(21) Anmeldenummer: 92107789.7
(22) Anmeldetag: 08.05.1992
(51) Int. Cl.: A61B 17/39, A61M 1/00

(54) **Koagulationssaug- und -spülinstrument**
Coagulation suction and irrigation device
Dispositif d'aspiration et d'irrigation de coagulation

(30) Priorität: 14.06.1991 DE 4119592
(43) Veröffentlichungstag der Anmeldung: 16.12.1992
(73) Patentinhaber: Richard Wolf GmbH, D-75438 Knittlingen (DE)
(72) Erfinder: Buess, Gerhard Fritz, Prof. Dr. med., W-7400 Tübingen (DE); Melzer, Andreas, Dr. cand. med., W-6200 Wiesbaden (DE); Boebel, Manfred, W-7136 Oetisheim (DE); Metsch, Dieter, W-7525 Kraichtal-Ba (DE)
(74) Vertreter: Vollmann, Heiko, Dipl.-Ing.

(56) Entgegenhaltungen:
- EP-A- 0 310 431
- WO-A-86/05379
- DE-A- 1 491 710
- DE-U- 9 114 124
- DE-U- 9 115 742
- US-A- 3 828 780

## Beschreibung

Die Erfindung geht aus von einem Koagulationssaug- und -spülinstrument mit einem einen Spül- und Saugkanal aufweisenden Schaft, um in eine Körperhöhle eine Spülflüssigkeit oder dergleichen zu- und abführen zu können, wie es im Oberbegriff des Anspruchs 1 definiert wird. Ein solches Instrument ist z.B. aus der EP-A-0 310 431 bekannt.

Bekannte Instrumente dieser Art sind so ausgebildet, daß deren Handhaben einen festen unlösbaren Teil des Instrumentes bilden. Bei diesen Instrumenten muß bei der Behandlung eines Patienten ein Instrumentenwechsel oder ein zweiter Einstich in den Körper des Patienten zu der Körperhöhle vorgenommen werden, wenn beispielsweise zusätzlich Hilfsinstrumente an den Ort des Eingriffes in der Körperhöhle heranzuführen sind.

In der DE-A-14 91 710 ist des weiteren ein Handgriff für ärztliche Zwecke beschrieben, an dem distal eine zweikanalige, in die Körperhöhle des Patienten einführbare Anordnung zum Spülen mit einer Flüssigkeit und Absaugen derselben und an dem proximal eine Zuführleitung und eine Abführleitung für die Flüssigkeit montierbar sind. Der Handgriff selbst besteht aus einem rohrartigen Außenteil mit zwei inneren Schlauchleitungen, die distale und proximale Anschlüsse an den im übrigen geschlossenen Enden des Außenteiles verbinden und der Zu- bzw. der Ableitung der Flüssigkeit durch den Handgriff hindurch dienen. Mit einer handbedienbaren Quetscheinrichtung werden die inneren Schlauchleitungen abgesperrt, um die Flüssigkeitsströmung durch den Handgriff hindurch zu unterbinden. Auch dieses Instrument ist nicht zum Durchführen zusätzlicher Hilfsinstrumente vorgesehen, noch ist es dazu geeignet. Es weist somit ebenfalls die vorstehend erwähnten Nachteile auf.

Die Aufgabe der Erfindung besteht daher darin, bei schwierigen endoskopischen Eingriffen, insbesondere auf engstem Raum, Hilfsinstrumente ohne Instrumentenwechsel und ohne zweiten Einstich an den Ort des Eingriffes heranführen zu können und die Handhabung des Instrumentes ergonomisch zu gestalten. Diese Aufgabe wird durch den Anspruch 1 gelöst.

In Weiterbildung des erfindungsgemäßen Instrumentes kann dieses von Rechts- und von Linkshändern ohne konstruktive Änderungen des Instrumentes verwendet werden. Dies wird durch die Merkmale des Anspruches 2 erreicht.

Weitere vorteilhafte Ausbildungen des Instrumentes sind in den Ansprüchen 3 bis 7 angegeben.

Die Erfindung wird nachstehend anhand der Zeichnungen erläutert. Es zeigt:

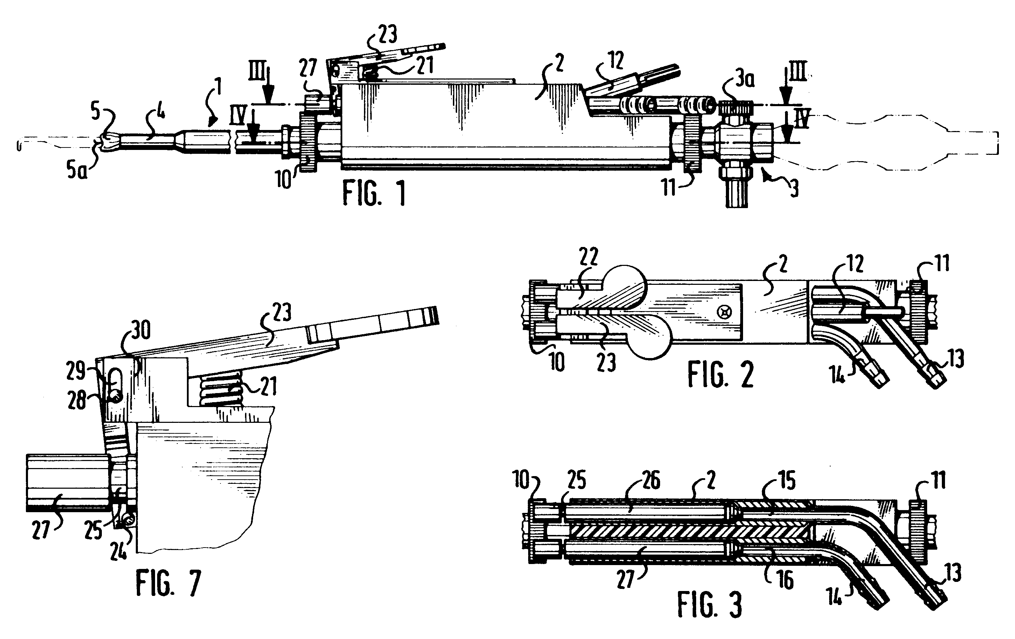

- Figur 1: eine Seitenansicht des Instrumentes nach der Erfindung,
- Figur 2: eine Aufsicht auf den Handhabenteil des Instrumentes nach Figur 1,
- Figur 3: einen Längsschnitt durch den Handhabenteil gemäß der Schnittlinie III-III in Figur 1,
- Figur 4: einen im Verhältnis in den Figuren 1 bis 3 etwas vergrößerten Längsschnitt durch die Handhabe nach der Schnittlinie IV-IV in Figur 1,
- Figur 5: eine Ansicht des Schaftes mit teilweisem Längsschnitt,
- Figur 6: einen Querschnitt nach Linie VI-VI der Figur 5,
- Figur 7: eine vergrößerte Seitenansicht des distalen Handhabenteiles mit Betätigungsmitteln für Ventile,
- Figur 8: einen Querschnitt nach Linie VIII-VIII der Figur 4 und
- Figur 9: einen vergrößerten Schnitt durch den distalen Teil des Instrumentenschaftes.

Nach der Erfindung besteht das Instrument zur Gewebekoagulation und zum Spülen mit Spülflüssigkeiten sowie zum Absaugen der Spülflüssigkeiten, Körpersekreten oder dergleichen aus drei Teilen 1,2 und 3, nämlich dem Instrumentenschaft 1 mit im Durchmesser verringertem distalen Ende 4 und der sich daran anschließenden Koagulalionselektrode 5 mit stirnseitig offenem, konkaven Ende 5a, der Handhabe 2 aus Isoliermaterial und dem mit einem absperrbaren Trompetenventil 3a versehenen Einführungsteil 3 für ein durch den Schaft 1 in die Körperhöhle zu führendes angedeutetes Hilfsinstrument.

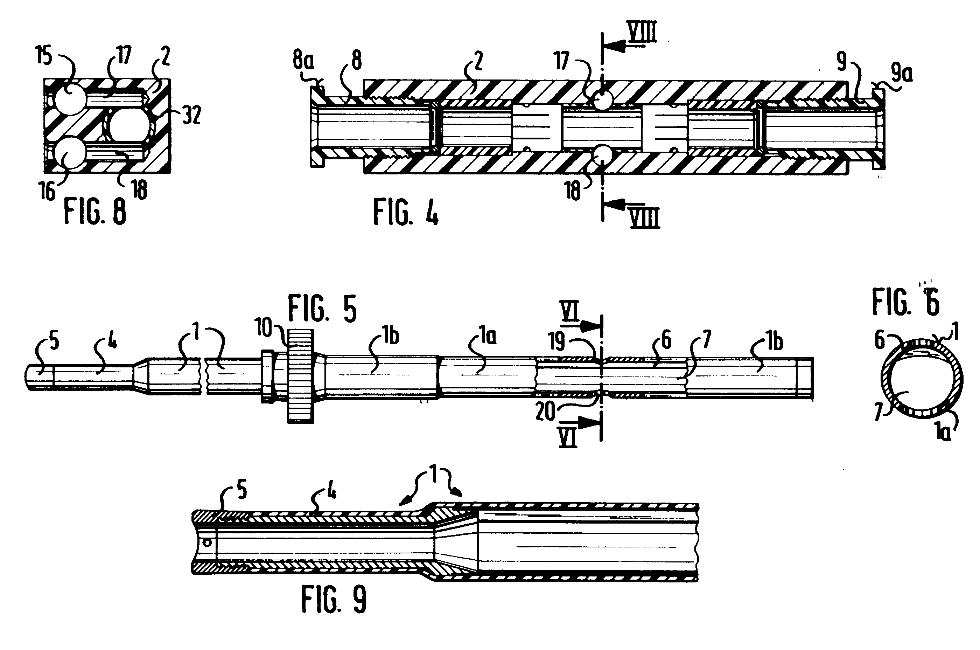

Der Schaft ist entsprechend der Figuren 5 und 9 in zwei Längskanäle 6 und 7 unterteilt, von denen der Kanal 6 zur Zufuhr von Spülflüssigkeit in die Körperhöhle und der im Querschnitt weitere Kanal 7 zur Durchführung eines Instrumententeiles und zum Abführen der Spülflüssigkeit, von Körpersekreten oder dergleichen dient.

Der Schaft 1 ist mit seinem proximalen Ende in den zentralen Kanal der Handhabe 2 eingeschoben und festgelegt. Zu diesem Zweck weisen beide Enden des den proximalen Schaftteil aufnehmenden Kanals der Handhabe Isolierhülsen 8 und 9 mit an den Enden angeordneten Gewinden 8a und 9a auf. Nach Einführen des proximalen Schaftendes in den Handhabenkanal wird der Schaft 1 durch einen auf das Gewinde 8a aufschraubbaren Schraubring 10 in der Handhabe festgelegt.

Der proximale Einführteil 3 wird durch einen auf das Gewinde 9a aufschraubbaren Schraubring 11 fest und sicher mit der Handhabe 2 verbunden. Der Rohrteil 1a ist auf dem Schaft 1 unlösbar zwischen den beiden zylindrischen Isolierhülsen 1b festgelegt.

Die Handhabe 2 ist auf der Oberseite mit einem HF-Anschluß 12 versehen, der mit dem nach außen isolierten Schaft 1 über Kontaktringe in der Handhabe 2 verbunden ist und durch den ein HF-Strom der distalen Koagulationselektrode 5 zugeführt wird. Die beiden Schaftkanäle 6 und 7 sind an Leitungen bzw. Anschlüsse 13,14 über Längskanäle 15,16 und hierzu senkrechte Kanäle 17,18 in der Handhabe 2 angeschlossen. Zu diesem Zweck sind die Wandungen der Schaftkanäle 6,7 mit Durchbrechungen 19,20 versehen, die sich an die Kanäle 17,18 anschließen, sobald das proximale Schaftende in die Handhabe eingeführt ist.

Zum Zu- und Abführen der Spülflüssigkeit sind auf der Handhabe 2 durch Fingerdruck gegen eine Feder 21 betätigbare, abgewinkelte Schaltwippen 22,23 gelagert, deren Arme einen Mitnehmerstift 24 besitzen, der in eine Ringnut 25 zweier Ventilstößel 26 und 27 eingreift. Durch Druck auf die Schaltwippe 22 wird der Ventilstößel 26 aus der Schließlage herausgezogen, und es kann dann eine Spülflüssigkeit über die Leitung 13 und die Kanäle 15,17 über die Durchbrechung 19 und den Schaftkanal 6 in die Körperhöhle geleitet werden. Durch Loslassen der Schaltwippe 22 wird das Durchströmen der Flüssigkeit automatisch unterbrochen, denn die Feder 21 drückt die Wippe zurück, und dabei wird gleichzeitig der Stößel 26 die Schließlage zurückgeschoben. Durch Betätigung der Schaltwippe 23 wird der Ventilstößel 27 betätigt und Spülflüssigkeit mit etwaigen Sekreten oder dergleichen über den Kanal 7 von der Saugleitung 14 aus der Körperhöhle abgenommen. Die Betätigung der Schaltwippen 22,23 bewirkt also eine Axialverschiebung der Stößel 26,27 und damit die Freigabe zum Absaugen der Spülflüssigkeit, und ein Loslassen der Schaltwippen bewirkt umgekehrt eine Absperrung der Kanäle.

Die beiden im Handhabenkanal festgelegten Isolierhülsen 8,9 besitzen von den Wanddurchbrechungen 19,20 bzw. den senkrechten Kanälen 17,18 gleichen Abstand. Dadurch kann der proximale Schaftteil entweder durch die Hülse 8 oder durch die Hülse 9 in den Schaftkanal eingeführt und fixiert werden, wodurch eine Anpassung der Handhabe an einen Rechts- oder Linkshänder möglich ist.

Der Schaftkanal 7 endet distal im Schaft 1 vor der distalen Schaftverengung. Dadurch besteht die Möglichkeit, das offene distale Schaftende gegen ein Körperorgan zur Anlage zu bringen und zu verschließen. Es kann nun die Spülflüssigkeit über den Kanal 6 zugeführt werden, die dann über den Kanal 7 vor der Schaftverengung abgesaugt wird, um dadurch den unter Umständen durch Sekrete oder dergleichen verlegten Kanal 7 freispülen zu können, ohne das Instrument aus der Körperhöhle nehmen zu müssen. Dieses Freispülen kann auch dann erfolgen, wenn durch den Kanal 7 ein Hilfsinstrument hindurchgeführt ist.

Durch die beschriebene Ausbildung des Instrumentes ist es möglich, die Teile 1,2 und 3 leicht und bequem zusammenzusetzen und auseinanderzunehmen, so daß eine einwandfreie Reinigung, Desinfektion und Sterilisierung aller Teile möglich ist. Zu diesem Zweck können auch die beiden Ventilstößel 26,27 aus der Handhabe 2 entnommen werden, indem der Mitnehmerstift 24 aus der Ringnut 25 entfernt wird, was dadurch möglich ist, daß jeweils das mittels des Stiftes 28 in der Kulisse 29 festgelegte Ende der Schaltwippe 22,23 im Winkel senkrecht zur Längsachse des Instrumentes so weit verschoben wird, bis der Stift 24 aus der Ringnut 25 herausgelangt ist, worauf diese Stellung durch ein nicht dargestelltes Kugelrastelement beispielsweise im Lager 30 der Schaltwippen 22,23 lösbar festgelegt werden kann. Befindet sich die Schaltwippe 22 oder 23 mit dem Stift 24 innerhalb der Kulisse 29 in der unteren Position (Figur 7), so wird durch die Kraft der Feder 21 der Stift 24 gegen die Handhabe 2 gedrückt, so daß durch diese Selbsthemmung auf zusätzliche Fixierelemente verzichtet werden kann.

Grundsätzlich besteht auch die Möglichkeit, den Schaft 1 um einen bestimmten Winkel um die Längsachse zu verdrehen, so daß dann der Kanal 7 an die Zufuhrkanäle 13,15,19 zum Freispülen des Kanals 7 angeschlossen wird.

Um auch an Körperröhren wie Blutgefäßen und dergleichen Koagulationen vornehmen zu können, ist das distale Ende der Elektrode 5 mit einer konkaven Ausnehmung 5a versehen, wodurch ein Abgleiten sicher vermieden wird.

## Patentansprüche

1. Koagulationsspül- und -sauginstrument mit einem einen Spül- und Saugkanal (6, 7) aufweisenden Schaft (1) zum Zu- und Abführen einer Spülflüssigkeit in eine und aus einer Körperhöhle, dadurch gekennzeichnet, daß das Instrument hauptsächlich aus folgenden drei lösbar verbundenen, Teilen besteht, nämlich einem Schaft (1) mit einem Spül- und einem Saugkanal (6, 7), einer auf dem proximalen Schaftende aufschiebbaren und festlegbaren Handhabe (2) aus Isoliermaterial mit HF-Anschuß (12) zum Schaft (1) und mit durch Fingerdruck freizugebenden Anschlußkanälen (13, 15, 17 und 14, 16, 18) zum Zu- und Abführen einer Spülflüssigkeit zu den beiden Schaftkanälen (6, 7) und einem proximal an den Schaft (1) und die Handhabe (2) ankuppelbaren, absperrbaren Einführteil (3) für durch den Saugkanal (7) des Schaftes (1) durchzuführende Hilfsinstrumente.

2. Instrument nach Anspruch 1, dadurch gekennzeichnet, daß in den beiden Enden des den proximalen Schaftteil aufnehmenden Kanals der Handhabe (2) für das Einführen und Festlegen des Schaftes eine zylindrische Isolierhülle (8, 9) befestigt ist, deren freies Ende ein Gewinde (8a, 9a) für einen Schraubring (10, 11) zum Festlegen des Schaftes (1) und zum Ankuppeln des Einführungsteiles (3) aufweist.

3. Instrument nach Anspruch 1 oder 2, dadurch gekennzeichnet, daß die beiden an eine Zufuhr- (13) und Abfuhrleitung (14) angeschlossenen Handhabenkanäle (15, 16) durch Kanäle (17, 18) mit den beiden Schaftkanälen (6, 7) verbunden sind und daß die Kanäle (15, 16, 17, 18) in ihrem Anschlußbereich durch axial verschiebbare Ventilstößel (26,27) mittels auf der Handhabe (2) gelagerter abgefederter Schaltwippen (22,23) zu öffnen sind.

4. Instrument nach einem der Ansprüche 1 bis 3, dadurch gekennzeichnet, daß zur Absperrung der beiden Handhabenkanäle (15,16) und der Schaftkanäle (6,7) an der Handhabe (2) zwei durch Fingerdruck gegen eine Feder (21) betätigbare Sehaltwippen (22,23) gelagert sind, welche Ventile in den Kanälen (15,17 und 16,18) öffnen und nach Loslassen der Schaltwippen schließen.

5. Instrument nach einem der Ansprüche 1 bis 4, dadurch gekennzeichnet, daß der zur Durchführung von Hilfsinstrumenten dienende Saugkanal (7) im Längenbereich des Instrumentenschaftes einen größeren Querschnitt als der Spülkanal (6) hat und distal vor dem im Querschnitt verkleinerten Schaftende (4) endet.

6. Instrument nach einem der Ansprüche 1 bis 5, dadurch gekennzeichnet, daß die Wandung der Schaftkanäle (6,7) mit zwei Durchbrechungen (19,20) zur Verbindung mit den Kanälen (17,15 und 18,16) versehen ist.

7. Instrument nach einem der Ansprüche 1 bis 6, dadurch gekennzeichnet, daß das distale, im Querschnitt verkleinerte Schaftende (4) eine abschließende Abflachung als Koagulationselektrode (5) aufweist.

8. Instrument nach einem der Ansprüche 1 bis 7, dadurch gekennzeichnet, daß in den Enden des das proximale Schaftende aufnehmenden Handhabenkanals Isolierhülsen (8,9) mit an ihren Enden vorgesehenen Gewindeenden (8a,9a) befestigt sind, die den gleichen Abstand von den Kanälen (17,18) und den Wanddurchbrechungen (19,20) des eingeführten Schaftendes aufweisen.

## Claims

1. Coagulation rinsing and suction instrument with a shaft (1) comprising a rinsing and suction channel (6, 7) for the delivery and removal of a rinsing liquid into and from a body cavity, characterised in that the instrument consists principally of the following three detachably connected parts, namely a shaft (1) with a rinsing and a suction channel (6, 7), a handle (2) made of insulating material with RF connection (12) to the shaft (1), which handle can be pushed open and fixed in position on the proximal end of the shaft and with connecting channels (13, 15, 17 and 14, 16, 18) to be released by finger pressure for the delivery and removal of a rinsing liquid at both shaft channels (6, 7), and an insertion part (3) which can be closed and can be coupled proximally to the shaft (1) and the handle (2) for auxiliary instruments to be led through the suction channel (7) of the shaft (1).

2. Instrument according to claim 1, characterised in that for the introduction and fixing of the shaft there is fixed in the two ends of the channel of the handle (2) receiving the proximal portion of the shaft, a cylindrical insulating casing (8, 9), the free end of which comprises a thread (8a, 9a) for a screw ring (10, 11) for fixing the shaft (1) and for coupling to the insertion part (3).

3. Instrument according to claim 1 or 2, characterised in that the two handle channels (15, 16) connected to a delivery (13) and removal channel (14) are connected by channels (17, 18) to the two shaft channels (6, 7) and that in their connecting region the channels (15, 16, 17, 18) are opened by axially displaceable valve tappets (26, 27) by means of sprung switching levers (22, 23) mounted on the handle (2).

4. Instrument according to one of the claims 1 to 3, characterised in that for closing the two handle channels (15, 16) and the shaft channels (6, 7), two switching levers (22, 23) which can be actuated by finger pressure against a spring (21) are mounted on the handle (2), which levers open valves in the channels (15, 17 and 16, 18) and close them after release of the switching levers.

5. Instrument according to one of the claims 1 to 4, characterised in that the suction channel (7) used for the passage of auxiliary instruments has a greater cross section in the longitudinal region of the instrument shaft than the rinsing channel (6) and terminates distally before the shaft end (4) of reduced cross section.

6. Instrument according to one of the claims 1 to 5, characterised in that the wall of the shaft channels (6, 7) is provided with two openings (19,20) for connecting to the channels (17, 15 and 18, 16).

7. Instrument according to one of the claims 1 to 6, characterised in that the distal end (4) of the shaft with reduced cross section comprises a final flattening as coagulation electrode (5).

8. Instrument according to one of the claims 1 to 7, characterised in that insulating sleeves (8, 9) provided with threaded ends (8a, 9a) at their ends are fastened in the ends of the handle channel receiving the proximal end of the shaft, which threaded ends are at the same distance from the channels (17, 18) and the wall openings (19, 20) of the introduced end of the shaft.

## Revendications

1. Instrument de balayage et d'aspiration pour coagulation comportant une tige (1) possédant un canal de balayage et d'aspiration (6,7) pour amener et évacuer un liquide de balayage dans une cavité corporelle et l'évacuer de cette cavité, caractérisé en ce que l'instrument est constitué principalement par trois parties reliées de façon amovible, indiquées ci-après, à savoir une tige (1) possédant un canal de balayage et un canal d'aspiration (6,7), une poignée (2) pouvant être emmanchée et fixée sur l'extrémité proche de la tige et réalisée en un matériau isolant et possédant une borne HF de raccordement à la tige (1), et des canaux de raccordement (13,15,17 et 14,16,18) qui doivent être libérés par la pression digitale et servent à amener un liquide de balayage aux deux canaux (6,7) de la tige, et une partie d'introduction (3), qui peut être bloquée et accouplée d'une manière proche à la tige (1) et à la poignée (2), pour des instruments auxiliaires que l'on doit faire passer dans le canal d'aspiration (7) de la tige (1), et évacuer le liquide de lavage des canaux de la tige.

2. Instrument suivant la revendication 1, caractérisé en ce que dans les deux extrémités du canal, qui loge la partie proche de la tige, de la poignée (2) est fixée, pour l'introduction et la fixation de la tige, une douille cylindrique isolante (8,9), dont l'extrémité libre comporte un filetage (8a,9a) pour une bague vissable (10,11) servant à fixer la tige (1) et à accoupler la partie d'introduction (2).

3. Instrument suivant la revendication 1 ou 2, caractérisé en ce que les deux canaux (15,16) de la poignée, qui sont raccordés à une canalisation d'amenée (13) et à une canalisation de conduite (14), sont reliés par des canaux (17,18) aux deux canaux (6,7) de la tige et que les canaux (15,16,17,18) doivent être ouverts, au niveau de leur zone de raccordement, par des pistons de soupape (26,27) déplaçables axialement, à l'aide de bascules de commutation (22,23) qui sont suspendues élastiquement et sont montées sur la poignée (2).

4. Instrument selon l'une des revendications 1 à 3, caractérisé en ce que pour la fermeture des deux canaux (15,16) de la poignée et des canaux (6,7) de la tige, sur la poignée (2) sont montées deux bascules de commutation (22,23), qui peuvent être actionnées au moyen de la pression digitale contre un ressort (21) et qui ouvrent des soupapes situées dans les canaux (15,17 et 16,18) et les ferment après relâchement des bascules de commutation.

5. Instrument selon l'une des revendications 1 à 4, caractérisé en ce que le canal d'aspiration (7), servant au passage des instruments auxiliaires, a, dans la zone allongée de la tige de l'instrument, une section transversale supérieure à celle du canal de balayage (6) et se termine, à distance, devant l'extrémité (4) de la tige, qui présente une section transversale réduite.

6. Instrument selon l'une des revendications 1 à 5, caractérisé en ce que la paroi des canaux (6,7) de la tige comporte deux ouvertures (19,20) pour la liaison avec les canaux (17,15 et 18,16).

7. Instrument selon l'une des revendications 1 à 6, caractérisé en ce que l'extrémité distale (4) de la tige, qui possède une section transversale réduite, possède un méplat terminal en tant qu'électrode de coagulation (5).

8. Instrument selon l'une des revendications 1 à 7, caractérisé en ce qu'aux extrémités du canal de la poignée, qui loge l'extrémité proximale de la tige, sont fixées des douilles isolantes (8,9), au moyen d'extrémités filetées (8a,9a), qui sont prévues sur les extrémités des douilles et sont situées à la même distance des canaux (17,18) et des ouvertures (19,20) aménagées dans la paroi de l'extrémité insérée de la tige.
